Europäisches Patentamt

European Patent Office

Office européen des brevets

(11) Numéro de publication: **0 101 373**
**B1**

(12) **FASCICULE DE BREVET EUROPEEN**

(45) Date de publication du fascicule du brevet: **03.10.90**

(51) Int. Cl.⁵: **A 61 M 1/38**

(21) Numéro de dépôt: **83401625.5**

(22) Date de dépôt: **08.08.83**

(54) Pancréas bio-artificiel à ultrafiltration.

(30) Priorité: **09.08.82 FR 8213869**

(43) Date de publication de la demande:
**22.02.84 Bulletin 84/08**

(45) Mention de la délivrance du brevet:
**03.10.90 Bulletin 90/40**

(84) Etats contractants désignés:
**AT BE CH DE FR GB IT LI LU NL SE**

(56) Documents cités:
**FR-A-2 242 129**
**FR-A-2 270 895**
**GB-A-1 479 002**
**US-A-3 827 565**
**US-A-4 013 564**
**US-A-4 242 460**

**ULLMANNS ENCYKLOPÄDIE DER
TECHNISCHEN CHEMIE, édition 4, vol. 16, 1978,
pages 528-529, Verlag Chemie, Weinheim, DE.**

(73) Titulaire: **CENTRE NATIONAL DE LA
RECHERCHE SCIENTIFIQUE (CNRS)
15, Quai Anatole France
F-75700 Paris Cedex 07 (FR)**

(72) Inventeur: **Jaffrin, Michel
4, rue Roger Couttelenc
F-60200 Compiegne (FR)**
Inventeur: **Reach, Gérard
14, rue Broca
F-75005 Paris (FR)**

(74) Mandataire: **Fort, Jacques
CABINET PLASSERAUD 84, rue d'Amsterdam
F-75009 Paris (FR)**

Courier Press, Leamington Spa, England.

**Description**

L'invention concerne les pancréas bio-artificiels, c'est-à-dire les dispositifs capables de réguler la glycémie d'un sujet diabétique, dispositif comportant des îlots de Langerhans (éventuellement provenant d'un être d'autre nature que le porteur du pancréas) destinés à sécréter l'insuline et protégés contre le rejet immun par une membrane perméable au glucose et à l'insuline, mais imperméable aux immunoglobulines et aux immunocytes.

Un pancréas bio-artificiel, qu'on désignera par la suite par l'abréviation PBA, est d'autant plus satisfaisant que sa réponse à une variation de concentration du glucose dans le sang se rapproche de celle du pancréas biologique. Il faut en particulier que l'insuline soit sécrétée par les îlots et parvienne au flux sanguin avant que la glycémie n'atteigne une valeur considérée comme le maximum acceptable et ce quelle que soit l'allure de la courbe d'augmentation de glycémie à partir du taux normal.

On a déjà fait de nombreuses études visant à la réalisation d'un PBA ayant une réponse cinétique acceptable. On trouvera notamment un résumé de ces études dans l'article de G. Reach et al dans "Journées Annuelles de Diabétologie de l'Hôtel-Dieu", Flammarion, Paris, 1982, pp. 147—159.

Dans tous les cas, le principe du PBA est le même: les îlots de Langerhans sont séparés de l'hôte diabétique par une membrane artificielle bio-compatible perméable au glucose et à l'insuline. Mais deux types de transfert du glucose aux îlots de Langerhans pour fournir une excitation à ces derniers ont été proposés et conduisent à des membranes de nature différente.

Un premier type de membrane utilise un phénomène de diffusion, c'est-à-dire de traversée de la membrane par les solutés eux-mêmes. Il s'agit d'un processus de dialyse, dont l'analyse montre qu'il est peu satisfaisant, car il exige un gradient de concentration, ce qui retarde la stimulation des îlots de Langerhans, et s'effectue à des vitesses différentes pour le glucose et l'insuline, ce qui trouble la fonction de transfert d'une boucle de régulation entre les variations glycémiques et la délivrance d'insuline dans le sang.

Un autre type de membrane utilise surtout un phénomène de convection, c'est-à-dire de traversée de la membrane par le solvant qui entraîne la masse des solutés dissous. Les membranes utilisables pour ce type de transfert sont des membranes ultrafiltrantes, retenant les molécules très lourdes. Dans ce cas, aucun gradient de concentration n'est nécessaire, ce qui est un facteur favorable, et la vitesse de transfert est déterminée par le gradient de pression hydrostatique à la traversée de la membrane et la perméabilité hydraulique de cette dernière.

On a déjà tenté de réaliser un PBA utilisant une membrane ultrafiltrante. On connaît en particulier (US—A—4 242 460) un PBA dans lequel les cellules sont disposées entre les spires jointives d'hélices parcourues en sens inverse par le flux sanguin et contenues dans un boîtier cylindrique. Cette disposition en double hélice fait que seule une faible fraction de la paroi filtrante des membranes en hélice est utilisée (celle qui borde les canaux étroits délimités par deux spires adjacentes) et seule une faible partie du volume dans lequel sont placés les îlots est parcourue par le courant convectif. Les îlots qui ne se trouvent pas dans cette partie restent inutilisés.

On connaît également un pancréas bio-artificiel conforme au préambule de la revendication 1 (voir GB—A—1479002), utilisant un tube constitué en une membrane microporeuse et enroulé en spirale dans un volume occupé par des îlots de Langerhans. La pression dans ce volume s'établit à une valeur moyenne entre les pressions du sang à l'entrée et à la sortie du tube. En conséquence, pour toute une partie du volume, la zone occupée par des îlots de Langerhans est séparée par la membrane microporeuse d'un flux sanguin qui est des deux côtés, soit supérieur à la pression dans le volume, soit inférieur à la pression dans le volume. Si la membrane est du type ultra-filtrante, le transfert d'insuline dans le flux sanguin ne peut se faire directement. Dans le second cas, le transfert de glucose depuis le flux sanguin se fait mal.

Par ailleurs, certains PBA qui ont une réponse satisfaisante en cas d'augmentation brutale de la glycémie (obtenue par exemple au cours d'essais, par injection intraveineuse) ne permettent pas de maintenir la glycémie au-dessous de la limite tolérable en cas de variation lente, par exemple à la suite d'un repas.

L'invention vise à écarter cet inconvénient et à fournir un PBA (1) ayant une réponse améliorée et permettant en particulier de maintenir la glycémie à un taux acceptable quelle que soit sa loi de variation dans le temps (2) faisant usage de la majeure partie de la surface filtrante et des îlots mis en oeuvre et (3) permettant de n'utiliser que la pression artérielle pour la production de l'ultrafiltrat, à l'exclusion de toute pompe exogène.

Dans ce but, l'invention propose un pancréas bio-artificiel du type ci-dessus défini, caractérisé en ce que les deux surfaces appartiennent à deux membranes ultra-filtrantes délimitant de deux côtés opposés la totalité dudit volume dans lequel sont contenus les îlots de Langerhans, en ce que les membranes sont ultra-filtrantes et en ce que le trajet du flux sanguin présente entre les deux tronçons un étranglement provoquant une perte de charge conduisant à des transferts d'ultra-filtrat du flux vers le volume dans le premier tronçon, du volume vers le flux dans le second tronçon.

Un tel PBA, où les îlots occupent un volume plat, est susceptible d'être réalisé avec des membranes de natures très diverses. On peut en particulier utiliser des membranes planes, par exemple en polyacrylonitrile tel que celui vendu sous la référence "AN 69" par Rhone Poulenc. On peut également utiliser une nappe de fibres creuses ultrafiltrantes. Les géométries adoptables sont également très diverses, comme on le verra plus loin.

Dans la pratique, le volume $V_1$ occupé par les

îlots de Langerhans et délimité par les membranes devra répondre à la condition

$$Vi \leqslant S \cdot P_M \cdot \tau \Delta P / 8$$

dans laquelle:

$\tau$ est la constante de temps acceptable, dont des études physiologiques effectuées sur l'homme indiquent qu'elle ne doit pas dépasser 20 minutes et doit, de préférence, être de 15 minutes au plus,

$\Delta P$ est la différence de pression motrice disponible, de l'ordre de 80 mm de mercure dans le cas courant d'une implantation en shunt artério-veineux,

S est la surface totale de la membrane,

$P_M$ est la perméabilité hydraulique de la membrane.

L'invention sera mieux comprise à la lecture de la description détaillée qui suit, qui se réfère aux dessins qui l'accompganent, dans lesquels:

la Figure 1 est un schéma montrant la constitution de principe d'un pancréas bio-artificiel et les grandeurs qui interviennent dans son fonctionnement;

la Figure 2 donne les courbes représentatives de la glycémie $G_B$ dans le sang et de la concentration en glucose $G_i$ dans le compartiment contenant les îlots d'un PBA, en fonction du temps, en réponse à une absorption de sucre;

la Figure 3 est un schéma de principe en coupe montrant un PBA à fibre creuse;

les Figures 4A et 4B montrent la disposition d'une fibre dans une variante de réalisation;

la Figure 5 montre un boîtier de PBA à membrane plate;

la Figure 6 est une vue de détail schématique montrant la mise en place des îlots de Langerhans dans une trame;

la Figure 7 montre une autre variante encore de réalisation, utilisant deux membranes ultrafiltrantes, en coupe suivant un plan perpendiculaire au sens de circulation du flux sanguin;

la Figure 8 est une vue de détail à grande échelle montrant le sac de la Figure 7.

Avant de décrire des modes particuliers d'exécution de l'invention, il peut être utile de rappeler quelques indications sur les conditions que doit remplir un PBA, puis de fournir une analyse d'un modèle de PBA.

Le principe d'un PBA est le suivant: un tissu pancréatique est placé dans un compartiment 10 séparé par une membrane ultrafiltrante 11 d'un flux sanguin de l'hôte diabétique, flux schématisé par une flèche 12. La membrane est traversée par un flux d'ultrafiltrat QF1 dans le sens sang-îlots et, dans sa partie aval, par un flux d'ultrafiltrats QF2 dans le sens îlots-sang. On appellera constante de temps $\tau$ la valeur $V_i/QF1$ ($V_i$ étant le volume du compartiment 10). On verra que cette constante de temps correspond au retard de l'insulino-sécrétion par le PBA, par rapport au pancréas endocrine normal, pour des variations très lentes de la glycémie $G_B$, par exemple à la suite d'un repas. A la suite d'une injection de glycémie, $G_B$ et

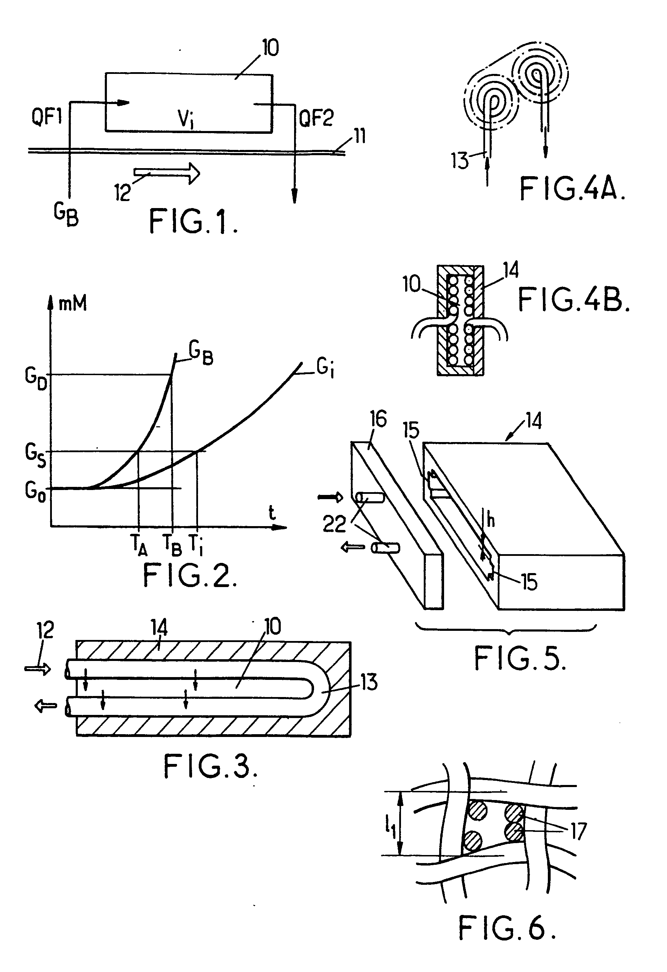

$G_i$ s'élèvent, suivant une loi du genre représenté en Figure 2, à partir de la glycémie $G_O$ à jeun. Les îlots de Langerhans commencent à sécréter de l'insuline dès que la glycémie atteint un seuil d'insulino-sécretion $G_S$. Dans le cas du pancréas biologique, la sécrétion commence à l'instant $T_A$ pour lequel $G_B = G_S$. Dans le cas d'un PBA, la sécrétion commence à l'instant $T_i$ pour lequel $G_i = G_S$. Si l'on désigne par $T_B$ l'instant auquel la glycémie atteint la limite supérieure acceptable $G_D$, une condition nécessaire pour que le PBA soit efficace et que l'on ait:

$$T_i \leqslant T_B$$

Ce n'est que dans ce cas que le PBA assure une administration d'insuline, régulée par la glycémie, avec un retard suffisamment faible pour la maintenir dans des limites acceptables.

Il faut au surplus tenir compte non seulement du retard à l'insulino-sécrétion, qui n'intervient qu'une fois que la concentration en glucose dans le compartiment 10 a atteint le seuil $G_s$, mais aussi du temps nécessaire au transfert de masse de l'insuline secrétée à travers la membrane 11. On verra que, dans un PBA suivant l'invention, la courte longueur du trajet suivi par le flux QF2, associée aux caractéristiques favorables de la membrane ultrafiltrante 11, permet de négliger le temps nécessaire au trasnfert de masse.

En utilisant le modèle de la figure 1, on peut déterminer par le calcul la relation qui existe entre la vitesse $\alpha$ de variation de la glycémie dans le sang, la constante de temps $\tau$ déjà définie et le retard $R = T_B - T_A$ à l'insulino-sécrétion. On trouve la relation:

$$\tau[\exp - \frac{(\alpha R + G_s - G_O)}{\alpha \tau} - 1] + R = 0$$

L'examen de cette formule montre que R tend vers $\tau$ lorsque $\alpha$ tend vers 0, c'est-à-dire que $\tau$ est le retard de l'insulino-sécrétion par le PBA, par rapport au pancréas normal, pour les variations lentes de la glycémie. Cette formule montre également que R dépend largement de $\alpha$ et que, pour qu'un PBA soit satisfaisant, il faut que R reste acceptable même dans le cas le plus défavorable où $\alpha$ est faible. Incidemment, la formule montre pour quelle raison divers PBA antérieurs améliorent la tolérance au glucose administré par voie veineuse, tandis qu'ils sont inefficaces pour combattre l'augmentation de la glycémie à la suite d'un repas. Dans le premier cas, $\alpha$ est très grand et on arrive à un retard R ne dépassant pas dix minutes. Au contraire, dans le cas d'un repas, $\alpha$ est faible et on arrive à une valeur de R dépassant quarante minutes. Or, l'expérience de l'insulino thérapie en boucle ouverte ou en boucle fermée (c'est-à-dire avec régulation) montre que le retard R ne doit pas dépasser vingt minutes et doit avantageusement rester inférieur ou égal à quinze minutes. En conséquence, un PBA répondant au modèle exposé ci-dessus devra remplir la condition:

$$V_i/QF1 < 20 \text{ min.}$$

(et de préférence à 15 min).

Cette condition impose un volume du compartiment 10 faible. Ce résultat ne peut être atteint qu'en adoptant des dispositions géométriques différentes de celles proposées jusqu'ici. De façon plus précise, on sera amené à disposer les îlots de Langerhans dans un volume mince, délimité par deux membranes ultrafiltrantes approximativement parallèles, balayées sur leurs surfaces externes par le même flux sanguin. La pression dans ce flux sanguin diminue le long du parcours du fait de la perte de charge répartie qu'il subit. Dans une première portion du parcours, où la pression du flux sanguin sera supérieure à la pression qui règne dans le compartiment 10 occupé par les îlots, il y aura passage du débit QF1 à travers la membrane filtrante. Dans la seconde portion, où la pression sanguine est inférieure à la pression dans le compartiment, il y aura passage du débit QF2 et transfert de masse de l'insuline vers le flux sanguin.

Les deux membranes ultrafiltrantes seront généralement constituées par deux portions successives d'une même membrane repliée en U sur elle-même. Cette membrane peut être soit plate, soit constituée par une ou des fibres creuses de diamètre interne suffisant pour éviter la coagulation.

Le schéma de la figure 3 montre la constitution de principe d'un PBA à une seule fibre 13 pliée en épingle à cheveux et parcourue par un flux sanguin. La fibre est placée dans un boîtier 14 et délimite le volume 10 occupé par les îlots. Du fait de la diminution de la pression hydrostatique par suite de la perte de charge le long de l'écoulement, le débit d'ultrafiltration par unité de longueur dans le compartiment 10 diminue depuis l'entrée jusqu'à une distance déterminée $L_1$, sensiblement égale à la moitié de la longueur totale L de la fibre, où il s'annule. Au-delà, il y a inversion de l'écoulement d'ultrafiltration et augmentation progressive du débit linéaire d'ultrafiltration jusqu'à la sortie.

Une étude théorique de ce montage de principe fait apparaître que la régulation permet d'éviter une hyperglycémie dépassant le seuil $G_B$ si la condition suivante est remplie:

$$V_i \leqslant \frac{S \cdot P_M \cdot \tau \cdot \Delta p}{8} \qquad (1)$$

Dans cette formule, $\tau$, $\Delta p$, S et $P_M$ ont la signification indiquée plus haut. La surface S à prendre en considération est évidemment uniquement celle qui délimite le compartiment 10.

Ainsi, pour une surface de membrane déterminée, de perméabilité hydraulique $P_m$, il existe un volume maximal du compartiment 10 permettant de conserver une valeur acceptable de $\tau$, typiquement 15 minutes, lorsque le PBA constitue un shunt artério-veineux, pour lequel $\Delta p$ est déterminé et de l'ordre de 80 mm de mercure. Le volume pourra être d'autant plus important que la perméabilité est plus élevée. A titre d'exemple, on peut indiquer que, dans le cas d'une implantation sur le rat utilisant une membrane Amicon XM50 de 50 cm de long et de 0,4 mm de rayon (au-dessous duquel on peut difficilement descendre sans risque de coagulation), le volume $V_i$ ne doit pas dépasser 200 µl. Le débit qui passe alors dans le PBA est compatible avec un shunt depuis la carotide sur le rat.

Il faut remarquer au passage que, grâce à la structure en épingle à cheveux de la fibre, le temps de retour de l'ultrafiltrat est très bref. Si les deux branches de la fibre sont séparées par une distance correspondant à l'épaisseur d'un îlot, c'est-à-dire 200 µm et qu'il se forme en moyenne 0,008 µl par minute dans le compartiment, le transfert de masse est réalisé en moyenne en une minute, ce qui est négligeable par rapport au temps de transmission de la variation du taux de glucose.

Une structure en épingle à cheveux simple présente l'inconvénient d'une longueur excessive pour permettre l'implantation. Diverses solutions permettent de s'affranchir de cette difficulté et en même temps de réduire les problèmes de courbure de fibre sous un faible rayon. Comme illustré en figures 4A et 4B, la fibre 13 est enroulée pour constituer deux limaçons successifs qui sont ensuite appliqués l'un contre l'autre en laissant simplement subsister l'épaisseur nécessaire pour intercaler les îlots, dans un boîtier 14 de forme plate. Avec deux fois sept spires, on peut réaliser un compartiment 10 ayant un volume de l'ordre de 100 µl, pouvant recevoir 800 îlots de 250 µm de diamètre. On retrouve toutefois dans ce montage le problème des courbures sous faible rayon lorsque la spire interne est de faible diamètre. Le problème est totalement écarté, mais au prix d'une diminution de compacité, en utilisant un montage en double solénoïde.

Dans un autre mode de réalisation de l'invention, on utilise une membrane plate flexible. Dans ce cas, la totalité de la surface de la membrane est utilisée pour délimiter le compartiment 10 sur la figure 3. Les îlots 17 étant situés entre les deux branches de la membrane, il convient d'adopter une disposition mécanique permettant de glisser l'ensemble des îlots et de la membrane dans le boîtier 14 (figure 5).

Une disposition commode, qui permet au surplus de diminuer le volume $V_i$, consiste à séparer les feuillets de la membrane par une trame en fil synthétique (polyamide ou polyester en général) délimitant des mailles où l'on place les îlots.

Quelques exemples numériques de réalisation seront maintenant donnés.

Pour réaliser un PBA utilisable pour des essais sur le rat, on peut adopter un encombrement en plan de 65×5,5 mm, donc une surface S de membrane de 7,15 cm²; pour permettre une épaisseur $e$ accrue du compartiment 10, une

trame de 300 µm d'épaisseur est placée entre les deux branches de la membrane (Figure 6). Cette trame maintient de plus l'écartement à une valeur déterminée. Si on utilise une membrane AN 69, fabriquée par Rhone Poulenc, dont la perméabilité $P_m$ est de 25 ml/h/m²/mmHg, on peut adopter une trame de pas $l_1 = 0,5$ mm, chaque maille recevant quatre îlots. Une autre solution, plus avantageuse, consiste à utiliser une membrane AN 69S ayant une perméabilité de 40 ml/h/m²/mmHg, avec une trame ayant un pas de 1 mm. Les 800 îlots nécessaires peuvent être placés uniquement dans les mailles du premier tiers de la trame, où la différence de pression hydrostatique entre les deux branches provoque un débit transmembranaire important. La différence de pression totale disponible sur un shunt artério-veineux ne dépassant pas 80 mm de mercure et les raccords se traduisant par une perte de charge qui peut atteindre 10 mm de mercure, l'épaisseur moyenne du film de sang se trouve approximativement déterminée. Pour un débit de 5 ml/mn, cette épaisseur sera de l'ordre de 200 µm. Si on dépasse cette épaisseur en maintenant le débit à la même valeur, il y aura une baisse du débit transmembranaire et donc une perte d'efficacité.

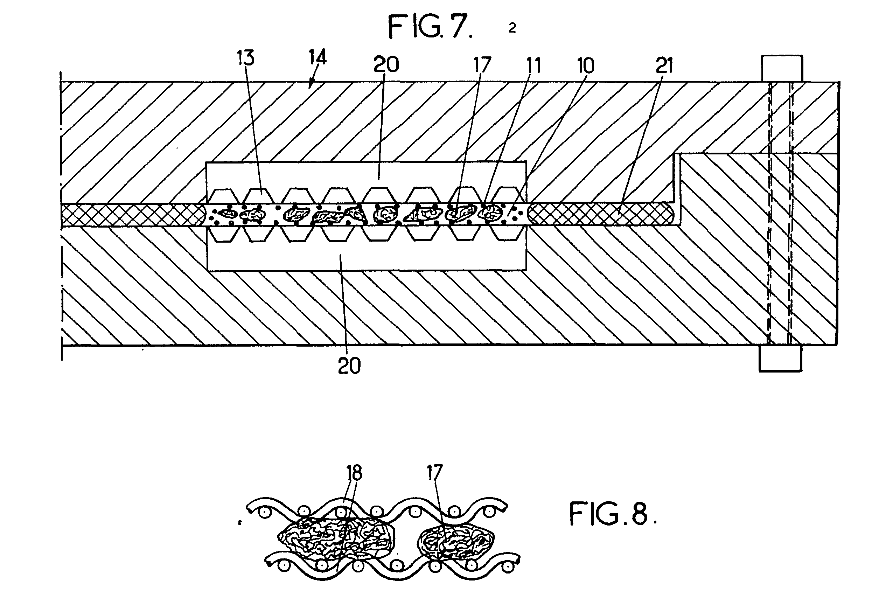

Une autre disposition, qui présente le grand avantage de faciliter la mise en place des îlots de Langerhans 17, est montrée en Figures 7 et 8. Les îlots sont placés dans un sac en toile 18 formée de fils en matériau thermosoudable pouvant servir de support de culture. On peut notamment utiliser un polyamide. Les mailles du sac doivent avoir une dimension telle que les îlots ne puissent s'échapper. Une maille inférieure à 70 µm entre fils de 30 µm donne des résultats satisfaisants.

Dans le cas montré en Figure 7, les canaux 13 parcourus par le flux sanguin sont délimités par une membrane en polyacronitrile 11 et des rainures ménagées dans des pièces 20 d'appui des membranes, retenues dans un boîtier 14 en deux pièces assemblées. Un joint d'étanchéité 21 en élastomère siliconé renforcé est interposé entre les deux pièces du boîtier. L'appui de la membrane soumise à une différence de pression tendant à l'écarter des îlots sur la pièce 20 correspondante assure la tenue mécanique. On peut adopter une épaisseur du compartiment 10 d'environ 360 µm et une profondeur de rainure de 400 µm. Plusieurs boîtiers de ce type peuvent être montés en parallèle.

Il faut encore remarquer que la partie de la membrane à proximité de l'extrémité du U ne joue guère de rôle pour les tranferts, surtout si le comaprtiment 10 ne contient pas d'îlots dans cette zone. On peut donc diminuer la longueur de la trame et de la membrane, ainsi que l'encombrement en plan du PBA, en augmentant localement la perte de charge près de l'extrémité du U. Cette augmentation peut être réalisée en diminuant l'épaisseur du film de sang ou sa largeur à cet endroit, par exemple à l'aide d'un étranglement qui doit toutefois être réalisé de façon à ne pas créer de risques de coagulation et d'obturation locales.

Un tel PBA peut être monté dans un boîtier du genre montré en Figure 5. La membrane est d'abord posée à plat, puis recouverte sur la moitié de sa longueur par la trame. Les îlots de Langerhans sont déposés dans les mailles de la trame, puis la membrane refermée sur la trame. L'ensemble ainsi constitué est alors enfilé dans le boîtier 14, muni de rainures latérales 15 de guidage et de maintien du sandwich ainsi formé. La rainure 15 présente une épaisseur correspondant à celle de la membrane et de la trame et les distances $h$ entre les bords des rainures et le fond de la cavité dans le boîtier fixent l'épaisseur des films de sang. Un couvercle 16, muni de moyens d'étanchéité non représentés, ferme ensuite le boîtier. Des raccords 22 prévus dans le couvercle permettent d'organiser la circulation.

Lorsque l'on souhaite extrapoler un tel PBA à l'organisme humain, il faut tenir compte du fait que le nombre d'îlots nécessaire atteint 200 000 environ. Mais la différence de pression artério-veineuse disponible n'est pas sensiblement modifiée, bien que le débit sanguin qui peut être détourné puisse être accru, pratiquement multiplié par 15 dans le cas d'une implantation sur le bras. La meilleure solution semble être alors de disposer les îlots en plusieurs couches en prévoyant une trame d'épaisseur plus forte ou plusieurs trames superposées. On peut ainsi envisager de réaliser un PBA à membrane plate ayant un encombrement en plan de 130×55 mm, ce qui permet de conserver un rapport longueur/largeur acceptable. La membrane délimite un film de sang de 320 µm d'épaisseur et le compartiment entre les deux plis de la membrane contient quatre couches d'îlots répartis dans les mailles de la trame.

D'autres dispositions encore sont possibles, par exemple sous forme bobinée, mais elles rendent plus difficile la mise en place dans un boîtier.

Il va sans dire que la présente invention ne se limite pas aux modes particuliers de réalisation donnés à titre d'exemples, mais s'étend à toute variante restant dans le cadre des équivalences.

**Revendications**

1. Pancréas bio-artificiel comportant des îlots de Langerhans contenus dans un volume limité par une membrane présentant des surfaces approximativement parallèles opposées au volume occupé par les îlots et successivement balayées par deux tronçons successifs du même flux sanguin sur une longueur supérieure d'au moins un ordre de grandeur à la distance qui sépare lesdites surfaces, caractérisé en ce que les deux surfaces appartiennent à deux membranes ultra-filtrantes délimitant de deux côtés opposés la totalité dudit volume, en ce que les membranes sont ultra-filtrantes et en ce que le trajet du flux sanguin présente entre les deux tronçons un étranglement provoquant une perte de charge conduisant à des transferts d'ultra-filtrat du flux vers le volume dans le premier tronçon, du volume vers le flux dans le second tronçon.

2. Pancréas bio-artificiel selon la revendication 1, caractérisé en ce que le volume $V_i$ occupé par les îlots de Langerhans et délimité par les membranes répond à la condition:

$$Vi \leqslant S \cdot P_M \cdot \tau\Delta P/8 \qquad (1)$$

dans laquelle:

$\tau$ est la constante de temps acceptable, dont des études physiologiques effectuées sur l'homme indiquent qu'elle ne doit pas dépasser 20 minutes,

$\Delta P$ est la différence de pression motrice disponible, de l'ordre de 80 mm de mercure dans le cas courant d'une implantation en shunt artério-veineux,

S est la surface totale de la membrane,

$P_M$ est la perméabilité hydraulique de la membrane.

3. Pancréas bio-artificiel selon la revendication 1 ou 2, caractérisé en ce que les membranes sont constituées par deux portions successives d'une même membrane plate repliée en U.

4. Pancréas bio-artificiel selon la revendication 3, caractérisé en ce que les deux parties de la membrane sont séparées par une trame définissant l'épaisseur du volume (10) et réduisant celui-ci.

5. Pancréas bio-artificiel selon la revendication 4, caractérisé en ce que les îlots de Langerhans sont disposés, en une ou plusieurs couches, dans les mailles de la trame.

6. Pancréas bio-artificiel selon la revendication 1 ou 2, caractérisé en ce que la membrane est constituée par au moins une fibre creuse ultrafiltrante repliée en U.

7. Pancréas bio-artificiel selon l'une quelconque des revendications 3 à 6, caractérisé en ce que le volume (10) ne contient des îlots de Langerhans (17) que dans la partie éloignée de l'extrémité du U.

8. Pancréas bio-artificiel selon l'une quelconque des revendications 3 à 7, caractérisé en ce que l'étranglement est placé à proximité de l'extrémité du U.

**Patentansprüche**

1. Biologisch-künstliches Pankreas, welches Langerhans-Inseln enthält, welche ihrerseits in einem Volumen enthalten sind, das von einer Membran begrenzt wird, welche Membran annähernd parallele Oberflächen aufweist, welche dem von den Inseln eingenommenen Volumen gegenüberliegen und nacheinander von zwei aufeinanderfolgenden Teilabschnitten ein und desselben Blutstromes über eine Länge bestrichen werden, welche um wenigstens eine Größenordnung größer als der Abstand ist, der die genannten Oberflächen voneinander trennt, dadurch gekennzeichnet, daß die beiden Oberflächen zu zwei ultrafiltrierenden Membranen gehören, welche von zwei gegenüberliegenden Seiten die Gesamtheit dieses Volumens begrenzen; daß die Membranen ultrafiltrierend sind, und daß die Bahn des Blutstromes zwischen den beiden Teilabschnitten eine Einschnürung aufweist, welche einen Druckverlust hervorruft, der wiederum einerseits zu einer Übertragung von Ultrafiltrat aus dem Blutstrom in Richtung auf das Volumen in dem ersten Teilabschnitt hin, und andererseits zu einer Übertragung aus dem Volumen in Richtung auf den Blutstrom hin, in dem zweiten Teilabschnitt führt.

2. Biologisch-künstliches Pankreas nach Anspruch 1, dadurch gekennzeichnet, daß das von den Langerhans-Inseln eingenommene und von den Membranen begrenzte Volumen $V_i$ der Bedingung:

$$Vi < S \cdot P_M \cdot \tau\Delta P/8 \qquad (1)$$

entspricht, worin

$\tau$ die Konstante einer annehmbaren Zeit ist, wobei am Menschen durchgeführte, diesbezügliche physiologische Untersuchungen zeigen, daß diese Konstante nicht größer als 20 min sein darf;

$\Delta P$ der verfügbare Unterschied im Steuerdruck ist, welcher für den geläufigen Fall einer Implantation als arteriovenöser Shunt in der Größenordnung von 80 mmHg ist;

S die Gesamtoberfläche der Membran ist; und

$P_M$ die hydraulische Durchlässigkeit der Membran ist.

3. Biologisch-künstliches Pankreas nach Anspruch 1 oder 2, dadurch gekennzeichnet, daß die Membranen aus zwei aufeinanderfolgenden Teilen ein und derselben flachen, U-förmig gefalteten Membran bestehen.

4. Biologisch-künstliches Pankreas nach Anspruch 3, dadurch gekennzeichnet, daß die zwei Teile der Membran voneinander durch ein Gewebe getrennt sind, welches die Dicke des Volumens (10) bestimmt und dasselbe verringert.

5. Biologisch-künstliches Pankreas nach Anspruch 4, dadurch gekennzeichnet, daß die Langerhans-Inseln in einer Schicht oder in mehreren Schichten in den Maschen des Gewebes angeordnet sind.

6. Biologisch-künstliches Pankreas nach einem der Ansprüche 3 bis 6, dadurch gekennzeichnet, daß die Membran aus wenigstens einer ultrafiltrierenden, U-förmig gefalteten Hohlfaser besteht.

7. Biologisch-künstliches Pankreas nach einem der Ansprüche 3 bis 6, dadurch gekennzeichnet, daß das Volumen (10) nur in dem von dem Ende des Buchstabens U entfernten Teil Langerhans-Inseln (17) enthält.

8. Biologisch-künstliches Pankreas nach einem der Ansprüche 3 bis 7, dadurch gekennzeichnet, daß die Einschnürung in der Nähe des Endes des Buchstabens U angeordnet ist.

**Claims**

1. Bio-artificial pancreas comprising islets of Langerhans contained in a volume defined by a diaphragm, having substantially parallel surfaces which are opposed to the volume receiving the islets and successively swept by two successive sections of a same blood flow over a length which is higher than the distance which separates said surfaces by at least one order of magnitude,

characterized in that the two surfaces belong to two ultrafiltration diaphragms which define the whole of said volume on two opposite sides, in that the diaphragms are ultrafiltration diaphragms and in that the path of the blood flow has, between the two sections, a restriction which causes a headloss leading to ultrafiltration transfers from the flow toward the volume in the first section, from the volume toward the flow in the second section.

2. Bio-artificial pancreas according to claim 1, characterized in that the volume $V_I$ occupied by the islets of Langerhans and defined by the diaphragms complies with the condition:

$$V_i \leqslant S \cdot P_M \cdot \tau \Delta P/8 \qquad (1)$$

in which:

$\tau$ is the acceptable time constant, physiological studies of which carried out on man indicate that it should not exceed 20 minutes;

$\Delta P$ is the available drive pressure difference, of the order of 80 mm of mercury in the general case of an arteriovenous shunt implant;

S is the total diaphragm area;

$P_M$ is the hydraulic permeability of the membrane.

3. Bio-artificial pancreas according to claim 1, characterized in that the diaphragms are formed by two successive portions of a same U-folded flat membrane.

4. Bio-artificial pancreas according to claim 3, characterized in that the two portions of the membrane are separated by a lattice defining the thickness of the volume (10) and reducing this latter.

5. Bio-artificial pancreas according to claim 4, characterized in that the islets of Langerhans are disposed in one or more layers in the meshes of the lattice.

6. Bio-artificial pancreas according to claim 1, characterized in that the diaphragm is formed by at least one U-folded ultrafiltrating hollow fiber.

7. Bio-artificial pancreas according to any one of claims 3—6, characterized in that the volume (10) only contains islets of Langerhans (15) in the portion distant from the end of the U.

8. Bio-artificial pancreas according to any one of claims 3—7, characterized in that the restriction is located proximate the end of the U.

FIG.1.

FIG.4A.

FIG.4B.

FIG.2.

FIG.5.

FIG.3.

FIG.6.

# FIG. 7.

# FIG. 8.